# EUROPÄISCHE PATENTANMELDUNG

(11) **EP 4 349 289 A1**
(43) Veröffentlichungstag der Anmeldung: **10.04.2024**
(21) Anmeldenummer: 22200337.8
(22) Anmeldetag: 07.10.2022
(51) Int. Cl.: A61B 18/14

(54) **SONDE MIT DISTALER SCHNEIDELEKTRODE**

(71) Anmelder: Erbe Elektromedizin GmbH, 72072 Tübingen (DE)
(72) Erfinder: ADLER, Marcus, 72393 Burladingen (DE); KARCHER, Felix, 72072 Tuebingen (DE)
(74) Vertreter: Rüger Abel Patentanwälte PartGmbB

(57) **Zusammenfassung**

Eine erfindungsgemäße Sonde (10) weist an ihrem distalen Ende eine Schneidelektrode (34) auf, die an einem Endstück (17) der Sonde (10) ausgebildet ist. Von dem Endstück (17) erstreckt sich ein Draht (28) über die gesamte Länge der Sonde (10) durch diese. Der Draht (28) kann zur Stromzufuhr zu dem Endstück (17) dienen. Erfindungsgemäß weist die Schneidelektrode (34) eine Verzweigungsstelle (399 auf, von der sich Elektrodenflächen (37, 38) in mindestens drei Richtungen weg erstrecken.

## Beschreibung

Die Erfindung betrifft eine Sonde insbesondere zur HF-Ablation von biologischem Gewebe.

Sonden zur HF-Ablation dienen beispielsweise der Devitalisierung von Gewebeabschnitten, zum Beispiel der Devitalisierung von in Gewebe (z.B. Lunge oder Leber) eingebetteten Tumoren oder dergleichen.

Die EP 3 788 974 A1, die EP 1 181 896 A1 und die US 2005/0010201 A1 veranschaulichen jeweils eine Ablationssonde der genannten Bauart mit auf einem schlauchartigen Grundkörper gehaltenen Elektroden, die mit dem Ausgang eines elektrischen Generators zu verbinden sind. Die Elektroden dienen, wenn die Ablationssonden in Gewebe eingestochen sind, zur Erwärmung des Gewebes durch Bestromung. Damit das unmittelbar in Kontakt mit den Elektroden stehende Gewebe nicht durch Austrocknung hochohmig wird, sondern feucht und somit niederohmig bleibt, werden die Elektroden gekühlt. Dazu ist in dem Lumen des Schlauchs einer solchen Ablationssonde eine Fluidzuführkapillare angeordnet, über die kühlendes Fluid austritt und die Elektroden kühlt.

Das Einstechen einer Sonde in biologisches Gewebe stellt insbesondere bei der Tumorbehandlung eine kritische Aufgabe dar.Insbesondere soll das Eindringen in verkapselter Tumore oder schwer durchdringbarer vorgelagerter Strukturen ermöglicht werden. Zudem soll ein kontrolliertes Einstechen ohne hohen Kraftaufwand ermöglicht werden, denn dieses könnte zum Durchstoßen des Tumors führen. Ein solcher Durchstoß hätte die Verletzung nachgelagerter Strukturen oder die Verschleppung von Tumorzellen zur Folge. Es soll auch vermieden werden, dass Tumorzellen beim Durchstechen des Tumors in gesundes Gewebe eingetragen werden.

Aus der US 6,454,727 B1 ist ein Biopsieinstrument bekannt, das an seinem distalen Ende außen mit einer bügelförmigen Elektrode versehen ist. Diese ist mit einer elektrischen Stromzufuhr verbunden und schneidet, wenn sie aktiviert wird, in das Gewebe ein.

Davon ausgehend, ist es Aufgabe der Erfindung, eine verbesserte Sonde zu schaffen.

Diese Aufgabe wird mit der Sonde nach Anspruch 1 gelöst:

Die Sonde weist einen flexiblen Schlauch auf, auf dem eine, zwei oder mehrere voneinander in Axialrichtung des Schlauchs distanzierten Elektroden angeordnet sein können. Vorzugsweise können sich solche Elektroden dabei um den gesamten Umfang des Schlauchs herum sowie über eine (zwischen ihrem distalen und ihrem proximalen Ende zu messende) axiale Länge erstrecken. Die Elektroden sind vorzugsweise flexibel ausgebildet, so dass sie engen seitlichen Krümmungen folgen können. Sie können dazu nach Art von Schraubenfedern ausgebildet sein.

Die Elektroden sind an elektrische Leitungen angeschlossen, die sich von der jeweiligen Elektrode bis zu dem proximalen Ende des Schlauchs erstrecken und dort über geeignete Anschlussmittel mit einem speisenden Generator verbindbar sind. Der speisende Generator ist vorzugsweise darauf eingerichtet, eine hochfrequente Wechselspannung bereitzustellen und somit hochfrequenten Strom abzugeben.

Der Schlauch umschließt ein Lumen, das sich von seinem proximalen Ende bis zu seinem distalen Ende erstreckt und dort abgeschlossen ist. An dem distalen Ende ist ein Endstück vorgesehen, das mit dem Schlauch fest verbunden ist und dessen Lumen verschließen kann. In dem Lumen ist eine Fluidlieferleitung angeordnet, die sich von dem proximalen Ende des Schlauchs in distaler Richtung bis zu den Elektroden erstreckt. An dem proximalen Ende kann die Fluidlieferleitung mit einer geeigneten Anschlusseinrichtung versehen sein, um mit einer zum Beispiel in dem speisenden Gerät angeordneten Kryofluidquelle verbunden zu werden. Das speisende Gerät kann somit die Kryofluidquelle und den Generator zur Bestromung der Elektroden in einem Gerät vereinen. Getrennte Ausführungen sind ebenfalls möglich. Solche separaten Geräte können über eine geeignete Schnittstelle, z.B. einen BUS zu einem Gerätesystem verbunden werden.

Das Endstück zum Verschluss des die Elektroden tragenden Schlauchs kann aus einem Isolationsmaterial, beispielsweise Kunststoff oder Keramik oder auch aus Metall bestehen. Insbesondere kann es auch aus einer Kombination eines elektrisch isolierenden Materials mit einem elektrisch leitfähigen Material bestehen. Elektrisch leitfähige Bereiche des Endstücks können zum Beispiel als Elektroden genutzt werden, um das Einstechen der Sonde in biologisches Gewebe zu unterstützen. Das Endstück weist insbesondere einen als Schneidelektrode dienenden Fortsatz auf, der unter Freilassung einer streifenförmigen distalen Endfläche von einem Isolator eingefasst ist. Die freiliegende Endfläche ragt vorzugsweise kaum oder nicht aus dem Isolator heraus. Die so gebildete Schneidelektrode unterstützt das Einstechen der Sonde in biologisches Gewebe und hält eine laterale Schädigung des Gewebes gering. Insbesondere ist es damit möglich, Gewebe zu durchstechen ohne dabei einen negative Einfluss auf die darauf folgende Ablation auszuüben. Es kann auch die Ausbildung eines zu großen Kogaulationssaums um die Einstichstelle vrmieden werden, der eine Steigerung der Impedanz des zu abladierenden Gewebes zur Folge hätte. Insbesondere ist es damit möglich, malignes Gewebe oder eine rigide Gewebestruktur zu durchstechen, ohne dabei vitales malignes Gewebe in gesundes Gewebe einzuschleppen oder infolge des Kraftaufwands beim Durchstoßen ungewollt gesundes Gewebe zu veerletzen.

Die distale Schneidelektrode der erfindungsgemäßen Sonde reduziert außerdem den mechanischen Widerstand beim Einstechen in Gewebe, insbesondere in kompaktes Tumorgewebe. Die Schneidelektrode kann das beim Einstechen vor ihr liegende Gewebe devitalisieren und durchtrennen, so dass sie leicht in das Gewebe eindringen kann. Weil dadurch wesentlich weniger Schubkräfte aufzubringen sind als beim rein mechanischen Einstechen ohne Schneidelektrode, ist es nun möglich, besonders die Sonden besonders flexibel zu gestalten. Dies erleichtert die weitere Verschlankung der Ablationselektrode, d.h. es können Sonden mit besonders geringem Durchmesser geschaffen werden. Außerdem können sehr enge Biegeradien erzielt werden, was das Spektrum der Einsatz- und Behandlungsmöglichkeiten erhöht.

Vorzugsweise ist das Endstück mit einem zugfesten Draht fest verbunden, der sich von dem Endstück durch das Lumen der Sonde bis zu deren proximalen Ende erstreckt und dort verankert ist. Der Draht dient der Aufnahme von Zugkräften beim Entfernen der Sonde aus einem Patienten, insbesondere aus seinem Gewebe. Der Draht entlastet den Schlauch von Zugkräften, sodass die Sonde in jedem Fall, auch bei Behandlungsproblemen beispielsweise infolge einer Anhaftung der Ablationselektroden im Gewebe, sicher geborgen werden kann. Der Zugdraht weist vorzugsweise eine hohe Rückstellkraft nach einer durch äußere Kraft induzierten Verformung z.B. durch Abwinkelung des Zugangssystems.

Das Endstück der Sonde kann aus einer elektrisch leitfähigen Keramik oder aus einem Metall, z.B. Edelstahl oder Hartmetall bestehen. Das Endstück kann ganz oder auch lediglich im Bereich seines Fortsatzes oder auch nur an der der als Schneidelektrode dienenden Endfläche mit einer Beschichtung, z.B. einer Silberschicht versehen sein. Dadurch lassen sich die Schneideigenschaften positiv beeinflussen.

Die distale Endfläche der Sonde kann eine Verzweigungsstelle aufweisen, von der sich mehrere Abschnitte weg erstrecken. Die Verzweigungsstelle kann axial zentriert angeordnet sein. Die schneidend wirkenden streifenförmigen Bereiche öffnen so in dem Gewebe einen Kanal, durch den sich die Sonde leicht und auch ohne Verschleppung von Gewebe schieben lässt.

Bei der erfindungsgemäßen Sonde können die Abschnitte einer Pyramidenform, einer Kegelform, einer Ellipsoidform oder einer Kugelform folgend angeordnet sein. Der durch die Schneidelektrode freigeschnittene Gewebekanal lässt sich so leicht aufweiten. Dies gilt insbesondere, wenn der Fortsatz der Sonde drei- oder mehrflügelig ausgebildet ist. Dazu kann der Fortsatz mehrere Flügel umfassen, die sich von einer Mittelachse vorzugsweise radial weg erstrecken.

Das Endstück der Sonde kann aus einem Isolator und einem Metallkörper bestehen, der an der distalen Endfläche frei liegt. Es ist aber auch möglich, dass der Isolator durch eine Beschichtung des Metallkörpers gebildet ist. Es kann sich dabei um eine elektrisch nichtleitende Beschichtung aus Keramik oder Kunststoff handeln, die Teile der distalen Stirnfläche des Endstücks bedeckt und dazwischen Abschnitte frei lässt, die die Schneidelektrode bilden.

Das Endstück der Sonde kann mit einer Schneidstromquelle verbindbar ausgebildet sein. Weiter können an oder auf dem Schlauch eine oder mehrere Elektroden angeordnet sein. Wenigstens eine dieser Elektroden kann mit einer Ablationsspannungsquelle verbindbar sein und so als Rückführungselektrode (Neutralelektrode) für den von der Schneidelektrode ausgehenden Strom dienen. Es ist aber auch möglich, eine am dem Patienten anderenorts angebrachte großflächige Elektrode als Neutralelektrode zu nutzen.

Zu der Erfindung gehört auch ein Verfahren zum Einführen einer Sonde in einen Tumor oder sonstiges Gewebe, bei dem die Sonde in Gewebe eingestochen wird, das einen behandlungsbedürftigen Bereich, z.B. einen Tumor, enthält. Beim Einstechen wird das Endstück mit einer Schneidspannung beaufschlagt. Dies erleichtert das Durchstechen des Gewebes, insbesondere festeren Gewebes. Die Sonde kann dabei feinfühlig positioniert werden, ohne die Verschleppung von Gewebe entlang des gebildeten Stichkanals befürchten zu müssen.

Die Sonde wird nach durchgeführter Behandlung nach der Ablation des Tumors durch den Stichkanal zurückgezogen. Zur Vermeidung der Verschleppung von Tumorzellen kann vorgesehen werden, die Spitzenelektrode und/oder die Ablationselektrod(en) beim Herausziehen mit einem speziellen Mode zu bestromen. Dadurch kann das den Stichkanal umgebende Gewebe nach der Ablation koaguliert werden.

Weitere Einzelheiten vorteilhafter Ausführungsformen der Erfindung sind Gegenstand von Unteransprüchen. In der Zeichnung ist ein Ausführungsbeispiel der Erfindung veranschaulicht. Es zeigen:
Figur 1 die erfindungsgemäße Sonde, angeschlossen an ein speisendes Gerät, in perspektivischer Prinzipdarstellung,
Figur 2 das distale Ende der Sonde, in Längsrichtung geschnitten mit Blick in sein Lumen,
Figur 3 ein Endstück für die Sonde nach Figur 1 oder 2, in perspektivischer Explosionsdarstellung,
Figur 4 das Endstück nach Figur 3, in teilweise perspektivischer Darstellung,
Figur 5 das Endstück nach Figur 3 mit Isolator, in aufgeschnittener Seitenansicht,
Figur 6 und 7 verschiedene Varianten des Isolators, in perspektivischer Ansicht,
Figur 8 ein Endstück mit Schneidelektrode und Taschen zur Aufnahme von Isolationsmaterial, in perspektivischer Darstellung
Figur 9 biologisches Gewebe mit eingestochener Öffnung ohne Sonde und
Figur 10 biologisches Gewebe mit eingestochener Öffnung mit Sonde.

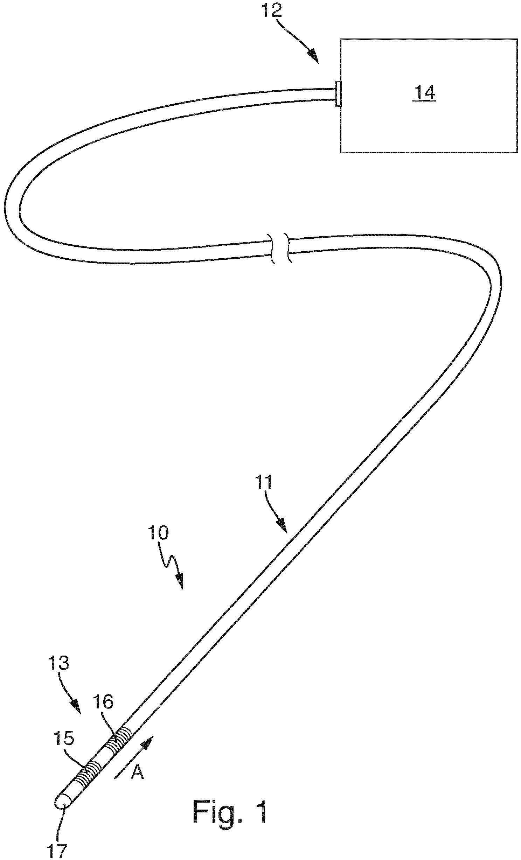

Figur 1 veranschaulicht eine Sonde 10, wie sie für den endoskopischen Einsatz, beispielsweise zur Devitalisierung von Tumoren oder anderem Gewebe (z.B. Lungentumoren), aber auch zu anderen Zwecken einsetzbar ist. Die Sonde 10 umfasst einen länglichen flexiblen Schlauch 11, der sich von einem proximalen Ende 12 bis zu dem distalen Ende 13 der Sonde 10 erstreckt. An dem proximalen Ende 12 sind ein oder mehrere Stecker oder eine andere geeignete Anschlusseinrichtung vorgesehen, um die Sonde 10 mit einem speisenden Gerät 14 zu verbinden.

Der Schlauch 11 ist vorzugsweise ein flexibler Kunststoffschlauch, der aus einem geeigneten bioverträglichen Kunststoff, beispielsweise PE/PET, PEEK oder ähnlichem gebildet ist. Während seine Länge mehrere Meter betragen kann, liegt der Durchmesser vorzugsweise im Bereich von 1 mm bis 3 mm. Andere Abmessungen sind je nach Anwendungsfall möglich und zweckmäßig.

Das distale Ende 13 der Sonde 10 kann eine oder mehrere Elektroden 15, 16 aufweisen, die in Axialrichtung A voneinander beabstandet sind. Die Elektrode 15 und gegebenenfalls vorhandene mindestens eine weitere Elektrode 16 können zur Gewebeablation dienen.

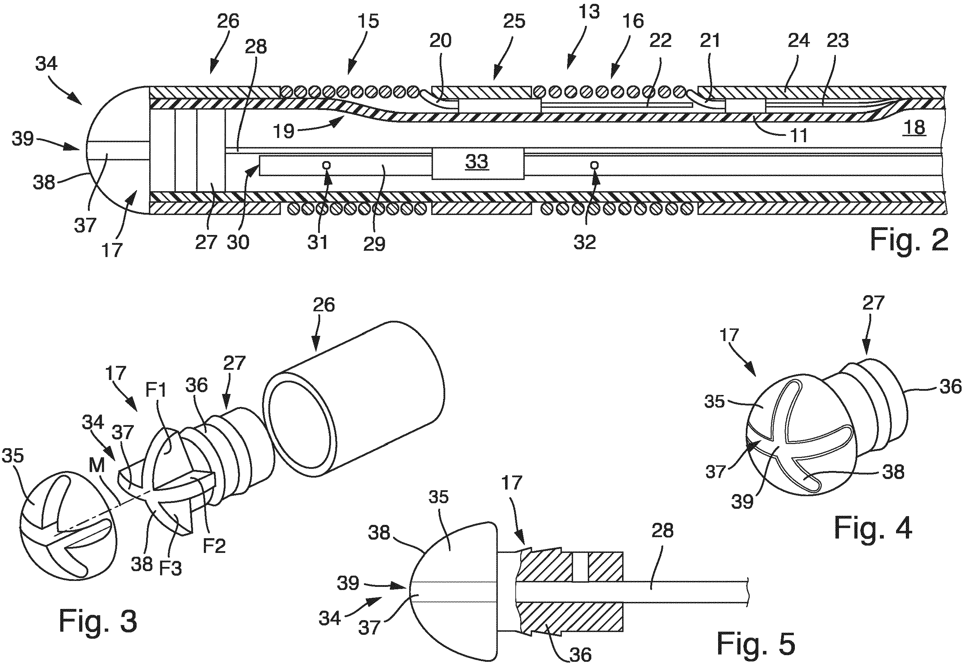

Der Schlauch 11 ist an seinem distalen Ende außerdem mit einem Endstück 17 versehen, das sein inneres, aus Figur 2 hervorgehendes Lumen 18 distal abschließt.

Der Schlauch 11 umschließt dieses Lumen 18 und ist dabei vorzugsweise lückenlos und unterbrechungsfrei ausgebildet. Die Elektroden 15, 16 sind auf dem Schlauch 11 im Bereich einer in den Schlauch 11 eingeformten Rinne 19 angeordnet. In der Rinne 19 liegen Anschlüsse 20, 21 der Elektroden 15, 16. Von den Anschlüssen 20, 21 erstecken sich elektrische Leitungen 22, 23 außen an dem Schlauch 11 entlang in proximaler Richtung zu dem Ende 12. Sie sind dort mit einem Stecker oder einer sonstigen Anschlusseinrichtung für das Gerät 14 verbunden.

Die Leitungen 22, 23 erstrecken sich durch einen Spalt zwischen dem Schlauch 11 und einem Überzugsschlauch 24, der vorzugsweise aus einem sehr flexiblen, gleitfähigen Kunststoff gebildet ist. Zwischen den Elektroden 15, 16 kann ein hülsenförmiger Isolator 25 angeordnet sein. Außerdem kann zwischen der distalen Elektrode 15 und dem Endstück 17 ein weiterer Isolator 26 angeordnet sein.

Das Endstück 17 ist an seiner distalen Außenfläche vorzugsweise gerundet. Zum Beispiel kann es halbkugelförmig ausgebildet sein. Von dem halbkugelförmigen Kopf erstreckt sich ein Schaft 27 in das Lumen, wobei der Schaft 27 formschlüssig und/oder mittels Klebstoff oder sonstiger geeigneter Befestigungsmittel an dem Schlauch 11 gesichert ist. Außerdem ist das Endstück 17 vorzugsweise mit einem Draht 28 verbunden, der sich von dem Endstück ausgehend bis zu dem beispielweise am proximalen Ende 12 der Sonde 10 angeordneten kraftübertragenden Teil der Sonde 10 erstreckt, um Zugkräfte von dem proximalen Ende sicher auf das distale Ende 13 zu übertragen. Der kraftübertragende Teil der Sonde 10 kann auch im Verlauf der Länge der Sonde 10 angeordnet sein.

In dem Lumen 18 kann eine Fluidlieferleitung 29 angeordnet sein, die als dünner Kunststoffschlauch, gegebenenfalls aber auch als Metallkapillare ausgebildet sein kann. Die Fluidlieferleitung 29 dient der Freisetzung von Kühlfluid an ihrem distalen Ende 30 oder einer oder mehreren seitlichen Öffnungen 31, 32. Das distale Ende der Fluidzuführleitung kann entsprechend offen oder, z.B. mittels Klebstoff oder mittels eines Pfropfens, geschlossen sein. Das Fluid kühlt die Elektroden 15, 16 und strömt dann durch das Lumen 18 zu dem Gerät 14 zurück. Es kann dort oder an dem proximalen Ende 12 der Sonde 10 ins Freie entlassen oder auch aufgefangen und recycelt werden.

Um die ausreichend genaue axiale Relativpositionierung der Öffnungen 31, 32 bezüglich der Elektroden 15, 16 sicherzustellen, kann die Fluidlieferleitung 29 an ihrem distalen Ende axial fixiert sein. Dazu kann sie z.B. mittels eines Schrumpfschlauchs 33, einer Klemme oder dergleichen mit dem Draht 28 verbunden sein. Dieser kann zum Beispiel aus einem Federstahl oder einem anderen besonders zugfesten Material, welches vorzugsweise eine hohe Rückstellneigung nach Verformung aufweist, wie zum Beispiel Nitinol, hergestellt sein. Auch ist die Verwendung eines Kunststoffdrahts möglich. Alternativ kann der Zugdraht 28 hohl ausgebildet sein und als Fluidlieferleitung dienen. Wie Figur 5 veranschaulicht, ist das Endstück 17 zugfest mit dem Zugdraht 28 verbunden, beispielweise verschweißt, verpresst und/oder verklebt. Insbesondere ist die Verbindung elektrisch leitfähig.

Das Endstück 17 ist aus den Figuren 3 und 4 gesondert ersichtlich. Es bildet eine Schneidelektrode 34 und kann dazu vollständig aus einem elektrisch leitfähigen Material, zum Beispiel als Metallkörper 36, als Hartmetallkörper aus Wolframkarbid, oder aus elektrisch leitfähiger Keramik ausgebildet sein. Das Endstück 17 kann mehrere (wenigstens drei) Flügel F1, F2, F3 aufweisen, die sich ausgehend von einer Mittelachse M vorzugsweise radial nach außen erstrecken und an denen die Schneidelektrode 34 ausgebildet ist.

Wie in den Figuren 3 und 4 veranschaulicht, kann dem Endstück 17 ein Isolator 35 zugeordnet sein, der nicht als Schneidelektrode dienende Bereiche des Endstücks 17 abdeckt und somit nach außen isoliert. Der Isolator kann die Flügel F1, F2, F3 aufnehmen.

Der Metallkörper 36 weist an seiner gerundeten Endfläche (z.B. an seinen Flügeln F1, F2, F3) Elektrodenflächen 37, 38 auf, die durch entsprechende Öffnungen des Isolators 35 ragen und die Schneidelektrode 34 bilden. Diese kann distal auch etwas über den Isolator 35 vorstehen. Der Metallkörper 36 kann dabei beispielsweise, wie in Figur 3 dargestellt, an seinem distalen Ende kreuzförmig ausgebildet sein. In diesem Fall gehen die Elektrodenflächen 37, 38 von einer zentralen Verzweigungsstelle 39 radial, dabei aber der etwa kugelförmigen Krümmung des distalen Endes des Verschlussstücks 17 folgend ab. Es ergibt sich eine vierflüglige Elektrodenstruktur, bei der die von der Verzweigungsstelle 39 abgehenden Elektrodenabschnitte miteinander paarweise Winkel von 90° einschließen. Die Elektrodenflächen 37, 38 gehen somit in vier verschiedenen Richtungen von der Verzweigungsstelle 39 ab. Es können aber auch dreiflüglige Elektrodenstrukturen mit Winkeln von 120° zwischen den Elektrodenflächen vorgesehen sein, so dass die Elektrodenflächen in drei Richtungen von der Verzweigungsstelle 39 (radial) nach außen abgehen. Auch fünf- oder mehrflüglige Strukturen können gewählt werden, wobei die Elektrodenflächen dann in fünf oder mehr Richtungen von der Verzweigungsstelle 39 (radial) nach außen abgehen.

Der Isolator 35 ist vorzugsweise aus einem Kunststoff, insbesondere aus einem gegen hohe Temperaturen beständigen und Kriechströmen widerstehenden Kunststoff oder Keramik gefertigt. Er weist eine Öffnung auf, die an die Form der Elektrodenflächen 37, 38 angepasst ist und deren Rand an diese anschließt. Der Isolator 35 kann wie in Figur 3 veranschaulicht als gesondertes Teil oder alternativ auch mit dem Isolator 26 einstückig ausgebildet sein, so dass diese ein gemeinsames einziges Teil bilden.

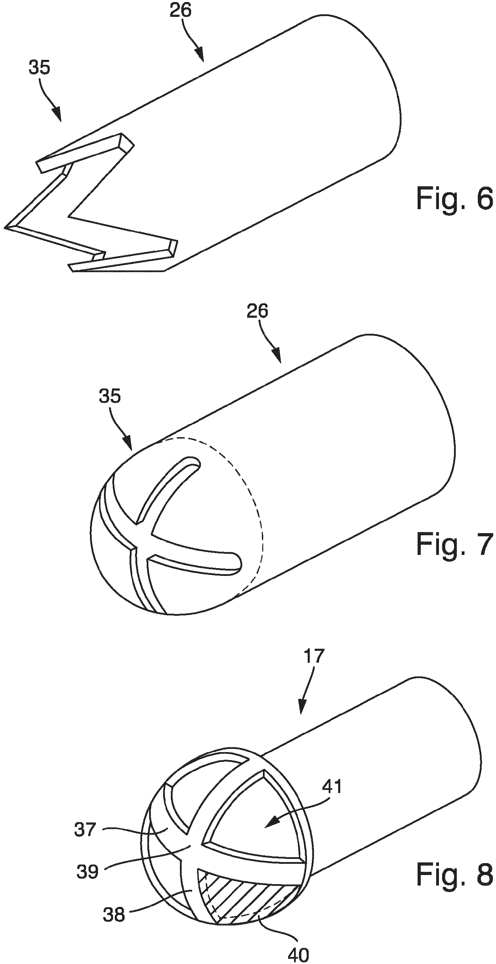

Solche Varianten der Isolatoren 26, 35 sind in den Figuren 6 und 7 veranschaulicht. Figur 6 veranschaulicht den Isolator 35, der als kronenartiger Fortsatz des hülsenförmigen Isolators 26 dargestellt ist. Nach dem Aufbringen des Isolators 26 auf das distale Ende des Schlauchs 11 können die vier Teile des in Figur 6 dargestellten Isolators 35 radial nach innen verformt werden, so dass der Isolator 35 die Gestalt nach Figur 7 annimmt. Der die Isolatoren 26, 35 umfassende Körper kann aber auch schon vor seiner Befestigung an der Sonde die in Figur 7 dargestellte Form aufweisen und, nachdem das Endstück 17 in das Ende des Schlauchs 11 eingebracht worden ist, in dieser Form auf das distale Ende des Schlauchs 11 aufgesetzt werden.

Weiter ist es möglich, den Isolator 35 durch isolierende Einlagen oder Beschichtungen 40 auszubilden, die in entsprechend geformten Vertiefungen oder Taschen 41 des Endstücks 17 angeordnet sind. Figur 8 veranschaulicht ein solches Endstück 17, bei dem diese Taschen 41 zwischen den metallisch blanken Elektrodenflächen 37, 38 ausgebildet sind. Die Einlagen oder Beschichtungen können aus Kunststoff, Keramik, Glas oder einem anderen elektrischen Nichtleiter bestehen. Wiederum können die Elektrodenflächen 37, 38 wie dargestellt eine vierflügliche Elektrode, alternativ aber auch eine dreiflüglige, fünfflüglige oder mehrflüglige Elektrode bilden. Die drei- und vierflüglige Variante wird aber als beste Ausführungsform angesehen.

Der die Zugkräfte aufnehmende Draht 28 kann an dem proximalen Ende 12 mit einer elektrischen Verbindungseinrichtung vorgesehen sein, um von dem Gerät 14 mit Spannung und Strom versorgt zu werden. Dadurch werden die Elektrodenflächen 37, 38 des Metallinlays 36 zum Beispiel beim Einstechen der Sonde 10 in biologisches Gewebe als Schneidelektrode wirksam.

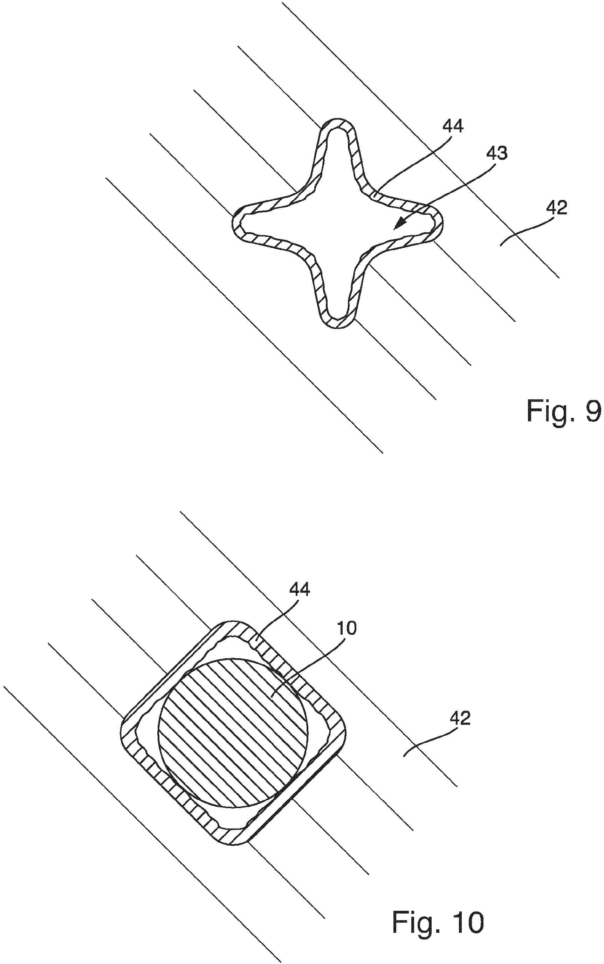

Die insoweit beschriebene Sonde 10 arbeitet wie folgt:
Die Sonde 10 wird allein oder über ein entsprechendes Zugangsinstrument, beispielsweise ein Endoskop oder, im Falle einer Ablation eines Lungentumors, ein Bronchoskop, in den Bronchialbaum des Patienten eingeführt. Die Sonde 10 wird dann in distaler Richtung aus dem Endoskop heraus weiter in distaler Richtung zu dem behandlungsbedürftigen Gewebe, beispielsweise dem Lungentumor, geschoben und in diesen eingestochen. Dazu wird die durch die Elektrodenflächen 37, 38 gebildete Schneidelektrode mit elektrischer Spannung beaufschlagt, deren Höhe zur Erzielung eines Gewebeschnitts ausreicht. Die Elektrodenflächen 37, 38 erzeugen in biologischem Gewebe 42 zunächst einen kreuzförmigen Schnitt 43 mit Koagulationsrand 44, wie in Figur 9 schematisch veranschaulicht. Beim Vorschieben der Sonde 10 wird der kreuzförmige Schnitt durch die Sonde 10 aufgeweitet, wobei die Sonde an den koagulierten Flächen des Koagulationsrands 43 entlang gleitet. Durch die glatte Außenform der Sonde und die elektrische Erzeugung des Schnitts 43 ist für das Einstechen der Sonde 10 in das Gewebe 42 nur eine geringe Vorschubkraft erforderlich. Die Sonde 10 kann dadurch sehr schlank und flexibel gestaltet werden. Außerdem verhindert die Koagulation des Gewebes 42 und die Ausbildung des Koagulationsrandes 43 ein Verschleppen von Gewebe in Einstechrichtung beim Durchstechen von Tumoren.

Nach dem Einstechen der Sonde 10 werden die Elektroden 15 und 16 mit Behandlungsspannung beaufschlagt und mit Behandlungsstrom versorgt. Zugleich oder kurz zuvor/nachgelagert wird die Fluidzufuhrleitung 29 mit Kühlfluid beaufschlagt, sodass dieses aus den Öffnungen 31, 32 austritt. Das Kühlfluid, beispielsweise Kohlendioxid, steht in der Fluidlieferleitung mit einem hohen Druck von einigen 10 Bar (Zum Beispiel 65 Bar) an und tritt durch die Öffnungen 31, 32 in das Lumen 18 über, in dem ein geringerer Druck von allenfalls wenigen Bar herrscht. Die Öffnungen 31, 32 dienen dabei als Drosselöffnungen, wobei durch den Joule-Thomson-Effekt Kälte erzeugt wird. Durch die feste Registrierung, d.h. axialer Ausrichtung der Öffnungen 31, 32 in Bezug auf die Elektroden 15, 16 wird die Kühlwirkung insbesondere etwa mittig zu den Elektroden 15, 16 erzeugt, sodass eine weitgehend gleichmäßige Kühlung jeder Elektrode 15, 16 sichergestellt ist. Damit wird eine zu große Erwärmung der Elektroden 15, 16 und somit ein Austrocknen des anliegenden Gewebes auch dann vermieden, wenn der Durchmesser der Sonde 10 im Wege der Miniaturisierung auf sehr geringe Werte von beispielsweise 2,3 mm oder weniger verringert wird, was zu hohen Stromdichten an den Elektroden 15, 16 führt. Mit dem erfindungsgemäßen Konzept ist jedoch eine Verbesserung und Vergleichmäßigung der Elektrodenkühlung zu bewirken, wodurch die Qualität der Ablationsbehandlung gefördert wird.

Es wird darauf hingewiesen, dass die Sonde 10 nicht nur, wie vorstehend beschrieben als Ablationssonde, sondern, ohne die Elektroden 15, 16, auch z.B. als Kryosonde oder ohne innere Kühlung auch als elektrische Nadel nur zum Einstechen in das Gewebe 42, d.h. zur Erzeugung eines mit einem Koagulationsrand 43 versehenen Kanals vorgesehen sein kann.

Eine erfindungsgemäße Sonde 10 weist an ihrem distalen Ende eine Schneidelektrode 34 auf, die an einem Endstück 17 der Sonde 10 ausgebildet ist. Von dem Endstück 17 erstreckt sich ein Draht 28 über die gesamte Länge der Sonde 10 durch diese. Der Draht 28 kann zur Stromzufuhr zu dem Endstück 17 dienen. Erfindungsgemäß weist die Schneidelektrode 34 eine Verzweigungsstelle 39 auf, von der sich Elektrodenflächen 37, 38 in mindestens drei Richtungen weg erstrecken.

### Bezugszeichen:

- 10: Sonde
- 11: Schlauch
- 12: proximales Ende der Sonde 10
- 13: distales Ende der Sonde 10 und des Schlauchs 11
- 14: Gerät/Geräteverbund, Schneidstromquelle, Ablationsspannungsquelle, Kühlfluidquelle
- 15: erste Elektrode
- 16: zweite Elektrode
- 17: Endstück
- 18: Lumen des Schlauchs 11
- 19: Rinne
- 20, 21: Anschlüsse der Elektroden 15, 16
- 22, 23: Leitungen
- 24: Überzugschlauch
- 25, 26: Isolator
- 27: Schaft
- 28: Zugdraht
- 29: Fluidlieferleitung
- 30: distales Ende der Fluidlieferleitung
- 31: erste seitliche Öffnung / Düsenöffnung
- 32: zweite seitliche Öffnung / Düsenöffnung
- 33: Schrumpfschlauch
- 34: Schneidelektrode
- F1 - F3: Flügel
- M: Mittelachse
- 35: Isolator
- 36: Metallkörper
- 37, 38: Elektrodenflächen
- 39: Verzweigungsstelle
- 40: Einlage/Beschichtung
- 41: Tasche
- 42: Gewebe
- 43: Schnitt
- 44: Koagulationsrand

## Patentansprüche

**1.** Sonde (10), insbesondere zur HF-Ablation,
mit einem flexiblen Schlauch (11), der an seinem distalen Ende (13) ein elektrisch leitendes Endstück (17) aufweist, das eine Schneidelektrode (34) aufweist, die unter Freilassung von streifenförmigen distalen Elektrodenflächen (37, 38) von einem Isolator (35) eingefasst ist.

**2.** Sonde nach Anspruch 1, **dadurch gekennzeichnet, dass** Endstück (17) aus einem Metall besteht.

**3.** Sonde nach einem der vorstehenden Ansprüche, **dadurch gekennzeichnet, dass** die distale Elektrodenflächen (37, 38) eine Verzweigungsstelle (39) aufweist, von der sie sich in mindestens drei verschiedenen Richtungen weg erstrecken.

**4.** Sonde nach Anspruch 3, **dadurch gekennzeichnet, dass** die Elektrodenflächen (37, 38) einer Pyramidenform, einer Kegelform, einer Ellipsoidform oder einer Kugelform folgend angeordnet sind.

**5.** Sonde nach einem der vorstehenden Ansprüche, **dadurch gekennzeichnet, dass** das Endstück (17) drei- oder mehrflügelig ausgebildet ist.

**6.** Sonde nach Anspruch 5, **dadurch gekennzeichnet, dass** das Endstück (17) mehrere Flügel (F1, F2, F3) umfasst, die sich von einer Mittelachse (M) weg erstrecken.

**7.** Sonde nach einem der vorstehenden Ansprüche, **dadurch gekennzeichnet**, das Endstück (17) aus einem Isolator (35) und einem Metallkörper (36) besteht, der an der distalen Endfläche frei liegt.

**8.** Sonde nach einem der vorstehenden Ansprüche, **dadurch gekennzeichnet, dass** der Isolator (35) durch eine Beschichtung des Metallkörper (36) gebildet ist.

**9.** Sonde nach einem der vorstehenden Ansprüche, **dadurch gekennzeichnet, dass** das Endstück (17) mit einem Draht (28) verbunden ist, der sich durch das Lumen (18) erstreckt.

**10.** Sonde nach einem der vorstehenden Ansprüche, **dadurch gekennzeichnet, dass** das Endstück (17) mit einer Schneidstromquelle (14) verbindbar ausgebildet ist.

**12.** Sonde nach einem der vorstehenden Ansprüche, **dadurch gekennzeichnet, dass** an oder auf dem Schlauch (11) mindestens eine Elektrode (15) angeordnet ist.

**13.** Sonde nach Anspruch 12, **dadurch gekennzeichnet, dass** die Elektrode (15) mit einer Ablationsspannungsquelle (14) verbindbar ist.

**14.** Sonde nach Anspruch 12 oder 13, **dadurch gekennzeichnet, dass** die Elektrode (15) zur Aufnahme eines von der Schneidelektrode (34) ausgehenden Stroms anschließbar ist.

**15.** Verfahren zum Einführen einer Sonde in einen Tumor nach einem der Ansprüche 1 bis 14, mit folgenden Schritten:
Einführen der Sonde (10) in Gewebe, das einen behandlungsbedürftigen Bereich, z.B. einen Tumor, enthält, dabei Spannungsbeaufschlagung des Endstücks (17) mit einer Schneidspannung,
Einstechen in den Tumor und dabei Positionieren mindestens einer Elektrode (15) in dem behandlungsbedürtigen Gewebe oder Durchstechen des Tumors mit der Schneidelektrode (34), bis diese in gesundes Gewebe trifft und dabei Positionieren mindestens einer Elektrode (15) in dem behandlungsbedürtigen Gewebe.
